Europäisches Patentamt

**European Patent Office**

**Office européen des brevets**

(19)

(11) Publication number: **0 012 514**
**B1**

(12) **EUROPEAN PATENT SPECIFICATION**

(45) Date of publication of patent specification: **16.02.83**

(21) Application number: **79302569.3**

(22) Date of filing: **14.11.79**

(51) Int. Cl.³: **C 07 C 15/00, C 07 C 2/66,**
**C 07 C 2/86, C 07 B 27/00,**
**C 07 C 21/24, C 07 C 17/26**

(54) Alkylation of aromatic hydrocarbons.

(30) Priority: **14.12.78 US 969630**

(43) Date of publication of application:
**25.06.80 Bulletin 80/13**

(45) Publication of the grant of the patent:
**16.02.83 Bulletin 83/7**

(84) Designated Contracting States:
**BE DE FR GB IT NL**

(56) References cited:
**GB - A - 1 525 423**
**US - A - 3 755 483**
**US - A - 4 049 737**
**US - A - 4 086 287**
**US - A - 4 094 921**
**US - A - 4 104 319**
**US - A - 4 112 056**

(73) Proprietor: **MOBIL OIL CORPORATION**
**150 East 42nd Street**
**New York New York 10017 (US)**

(72) Inventor: **Burress, George Thomas**
**80 Claire Drive**
**Bridgewater, New Jersey 08807 Somerset (US)**

(74) Representative: **Cooper, John Anthony et al,**
**Mobil Court 3 Clements Inn**
**London WC2A 2EB (GB)**

Courier Press, Leamington Spa, England.

## Alkylation of aromatic hydrocarbons

This invention is directed to the catalytic alkylation of aromatic hydrocarbons, in the liquid phase.

Alkylation of aromatic hydrocarbon compounds employing certain crystalline zeolite catalysts is known, for instance from U.S. 3,251,897 which describes liquid phase alkylation in the presence of crystalline aluminosilicates such as faujasite, heulandite, clinoptilolite, mordenite, dachiardite, zeolite X and zeolite Y. The temperature of such alkylation procedure does not exceed 315.55°C (600°F), thereby maintaining the operating phase substantially liquid.

U.S. 2,904,607 describes alkylation of hydrocarbon compounds in the presence of crystalline metallic aluminosilicates such as magnesium aluminosilicates.

U.S. 3,631,120 and 3,641,177 describe liquid phase processes for alkylation of aromatic hydrocarbons with olefins in the presence of certain zeolites. U.S. 3,631,120 discloses use of an ammonium exchanged, calcined zeolite having a silica to alumina mole ratio of between 4.0 and 4.9. U.S. 3,641,177 discloses use of a zeolite catalyst activated in a particular manner.

U.S. No. 3,755,483 discloses that the vapor phase reaction of propylene with benzene and toluene in the presence of zeolite ZSM-12 proceeds with high catalyst activity and selectivity to yield isopropylbenzene and isopropylmethylbenzene, but with operation in the vapor phase with relatively high temperatures and pressures, thereby promoting dealkylation and olefin decomposition which is especially evident when the alkylating agent comprises an olefin of three or more carbon atoms.

U.S. Patent 4,049,737 describes the selective production of cymene by vapor phase alkylation of toluene with propylene at 204 to 316°C in the presence of various crystalline aluminosilicate zeolites including ZSM-12. That process has the advantage that by operating at lower temperatures than the process of U.S. 3,755,483, better product selectivity is achieved.

A process has now been discovered whereby aromatic hydrocarbons, containing either polar or nonpolar substituents, may be successfully alkylated with alkylating agents containing from one to five carbon atoms. Surprising and substantial increases in product yield, conversion and selectivity are obtained by operating at generally lower temperatures than heretofore and essentially in the liquid phase. This increase is especially evident when alkylating agents of three or more carbon atoms (where higher temperatures promote dealkylation and olefin decomposition) are used.

According to the present invention a process for the liquid phase alkylation of an aromatic hydrocarbon compound comprises contacting such compound with an alkylating agent having from one to five carbon atoms at a temperature between 100°C and 300°C and a pressure at least sufficient to maintain a liquid phase but not greater than $2 \times 10^4$ kPa in the presence of a catalyst comprising zeolite ZSM-12.

The process is advantageously carried out at a temperature between 200°C and 250°C. A suitable aromatic/alkylating agent mole ratio is the range 20:1 to 1:1. The process is usually operated at a WHSV (with reference to total catalyst) of 0.1 to 100. The aromatic hydrocarbon may bear a polar or non-polar substituent, and the preferred alkylating agent is an olefin, particularly one having at least three carbon atoms. Alcohols, however, are also favoured alkylating agents.

The catalyst comprises zeolite ZSM-12. ZSM-12 is described and identified in terms of its X-ray data in U.S. Patent 3,832,449.

Zeolite ZSM-12, when prepared in the presence of organic actions, is substantially catalytically inactive, possibly because the intracrystalline free space is occupied by organic cations from the forming solution. It may be activated by heating in an inert atmosphere at 540°C for one hour, for example, followed by base exchange with ammonium salts followed by calcination at 540°C in air. The presence of organic cations in the forming solution may not be absolutely essential to the formation of this zeolite; however, the presence of these cations does appear to favor the formation of this zeolite. More generally, it is desirable to activate this zeolite by base exchange with ammonium salts followed by calcination in air at about 540°C for from about 15 minutes to about 24 hours.

When synthesized in the alkali metal form, the zeolite is conveniently converted to the hydrogen form, generally by intermediate formation of the ammonium forms as a result of ammonium ion exchange and calcination of the ammonium form to yield the hydrogen form. In addition to the hydrogen form, other forms of the zeolite wherein the original alkali metal has been reduced to less than about 1.5 percent by weight may be used. Thus, the original alkali metal of the zeolite may be replaced by ion exchange with other suitable ions of Groups IB to VIII of the Periodic Table, including, by way of example, nickel, copper, zinc, palladium, calcium or rare earth metals.

In practicing the desired conversion process, it may be desirable to incorporate the above described crystalline zeolite in another material resistant to the temperature and other conditions employed in the process. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, which families include the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the zeolite employed herein may be composited with a porous matrix material, such as alumina, silica-alumina, silica-magnesia, silica-zirconia, silica-thoria, silica-berylia, silica-titania as well as ternary compositions such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix on an anhydrous basis may vary widely, with the zeolite content ranging from between about 1 to 99 percent by weight and more usually in the range of about 5 to about 80 percent by weight of the dry composite.

Exemplary of the hydrocarbons which may be alkylated by the process of this invention are aromatic compounds such as benzenes, naphthalenes, anthracenes, and the like and substituted derivatives thereof; and alkyl substituted aromatics, e.g. toluene, xylene, and homologs thereof. In addition, other non-polar substituent groups may also be attached to the aromatic ring, including by way of example methyl, ethyl, t-butyl, alkyl, cycloalkyl, phenyl and aryl.

Aromatic hydrocarbons which may be alkylated by the present process likewise include those having polar substituents on the aromatic ring or on a side chain attached thereto. Non-limiting examples include phenol, cresols and halogenated aromatics.

The preferred alkylating agents are olefins, formaldehyde, alkyl halides and alcohols. Numerous other acyclic compounds having at least one reactive alkyl radical may be used.

The process may be carried out as a batch-type, semi-continuous or continuous operation subsequent to leaving the reaction zone, the desired products may be recovered from the reactor effluent, for instance by distillation, and the unreacted hydrocarbons recycled for reuse in the process.

The following examples illustrate the process of this invention without limiting the scope or utility thereof. The catalyst in all cases comprises 4.6 gms of ZSM-12 prepared as disclosed in U.S. 3,832,449 and consisted of 65 wt % HZSM-12 and 35 wt % $Al_2O_3$ binder. The reaction products were analyzed with a gas chromatograph equipped with a hot wire detector. Positive identification of the various compounds was made with GC/MS, IR, and/or NMR.

Examples 1—4

Benzene was alkylated with $C_3$ through $C_5$ alkenes in the presence of HZSM-12, at various temperatures and pressures. The reactions are summarized in Table I.

TABLE I
Benzene
Alkylation over HZSM-12

| Example | Alkylating agent | Temp.°C | Pressure | WHSV | Benzene/alkylating agent mole ratio | % Conversion alkylating agent | Products-% selectivity |
|---|---|---|---|---|---|---|---|
| 1 | Propylene | 200 | 2169.76 kPa (300 psig) | 6 | 7/1 | >95 | Isopropyl benzene-92% diisopropyl-benzene-7.5% |
| 2 | 1-Butene or 2-butene | 190 | 2514.49 kPa (350 psig) | 6 | 7/1 | >95 | Sec-butyl-benzene-95+% |
| 3 | Isobutylene | 190 | 2514.49 kPa (350 psig) | 6 | 7/1 | >95 | Tertiary butyl-benzene-95+% |
| 4 | 2-Methyl-butene-90% 2-Pentene-10% | 200 | 2514.49 kPa (350 psig) | 6 | 7/1 | >95 | Tertiary pentylben-zene-52% 1,2-di methyl propyl-benzene-38% |

**0012514**

The data illustrates the exceptional alkylation activity and selectivity of ZSM-12 catalyst at temperatures of 200°C and below with respect to alkylating agents $C_3$ and higher.

Examples 5—7

Toluene was alkylated with $C_3$ and $C_4$ olefins in the presence of HZSM-12 catalyst at a variety of temperatures and pressures. Table II is a summary of the reaction conditions and products formed.

TABLE II
Toluene
Alkylation over HZSM-12

| Example | Alkylating agent | Temp.°C | Pressure | WHSV | Toluene/alkylating agent mole ratio | % Conversion alkylating agent | Products-% selectivity |
|---|---|---|---|---|---|---|---|
| 5 | Propylene | 250 | 3548.7 kPa (500 psig) | 6 | 7/1 | >95 | Isopropyl-toluene-95% (32% *Para* 62% *Meta* 6% *Ortho*) |
| 6 | 1-Butene or 2-butene | 200 | 2169.76 kPa (300 psig) | 6 | 7/1 | >95 | Secondary Butyltoluene 95% (60% *Para* 40% *Meta*) |
| 7 | Isobutylene | 190 | 2169.76 kPa (300 psig) | 6 | 7/1 | >95 | Tertiary Butyltoluene 95% (60% *Para* 35% *Meta*) |

**0012514**

Toluene alkylation differs from benzene alkylation in that the initial alkylation product consists of isomers. Although the reactions with toluene can be made at the same temperatures as benzene, it was found necessary to vary the temperature in order to optimize the formation of a desired isomer. This is illustrated in Example 8 with toluene-propylene.

Example 8

Cymene (isopropyltoluene), the product of toluene-propylene alkylation, is of commercial interest as a large volume chemical intermediate for cresol manufacture. Of the three isomers, *meta* cymene has the greatest potential value and *ortho* cymene the least value. Because of the high potential for immediate commercial application, the reaction parameters of toluene-propylene alkylation with HZSM-12 were studied in depth to optimize *meta* cymene production.

The variable that most affected selectivity to *meta* cymene was found to be temperature. This is illustrated in the following Table III;

TABLE III
Toluene-propylene alkylation
HZSM-12 catalyst

| Temp.°C | Isopropyltoluene isomer distribution | | |
|---|---|---|---|
| | Ortho | Meta | Para |
| 200 | 18.4% | 31.8% | 49.8% |
| 230 | 6.9% | 60.3% | 32.8% |
| 260 | 5.3% | 63.7% | 31.0% |

WHSV-5.7 Toluene/0.4 $C_3H_6$
Pressure-3548.7 kPa (500 psig)
Molar Feed Ratio-6.25/1 Toluene/$C_3H_6$

Increasing the temperature above 260°C did not give a higher *meta* cymene content.

Propylene conversion at 230—240°C averaged 90—95% during a 5 day run. The only indication of a decrease in catalyst activity was a change in isomer distribution versus time on stream. This was denoted by a decrease in the *meta* isomer and an increase in the *para* and *ortho* isomers.

Examples 9, 10

Alkylation of ethylbenzene with butenes and isobutylene, respectively, in the presence of a ZSM-12 catalyst, is summarized in Table IV.

7

TABLE IV
Ethylbenzene
Alkylation over HZSM-12

| Example | Alkylating agent | Temp.°C | Pressure | WHSV | Ethylbenzene/alkylating agent mole/ratio | % Conversion agent | Products-% selectivity |
|---|---|---|---|---|---|---|---|
| 9 | 1-Butene or 2-butene | 200 | 2169.76 kPa (300 psig) | 6 | 7/1 | >95 | Secondary Butylethyl-benzene-90% (60% *Para*, 40% *Meta*) |
| 10 | Isobutylene | 200 | 2169.76 kPa (300 psig) | 6 | 7/1 | >95 | Tertiary Butylethyl-Benzene/-90% (90% *Para*, 10% *Meta*) |

0012514

**0012514**

Operating in the liquid phase, butene conversion was >95% with 90% selectivity to butylethylbenzene. 1-butene or 2-butene alkylated to produce secondary butylethylbenzene. The isomer ratio was 60% *para* and 40% *meta* at the temperature used for the experiment. No *ortho* was found.

At the same operating conditions, isobutylene alkylated to give identical olefin conversion and selectivity to product as was found with 1-butene or 2-butene. The isomer ratio of the tertiary butylethylbenzene was 90% *para* and 10% *meta*. This ratio is close to the 92/8 ratio obtained with conventional Freidel-Kraft catalysts, but conversion and selectivity are higher with HZSM-12.

Examples 11, 12

The alkylation of isopropylbenzene over HZSM-12 is summarized in Table V.

TABLE V
Isopropylbenzene
Alkylation over HZSM-12

| Example | Alkylating agent | Temp.°C | Pressure | WHSV | Isopropylbenzene/ alkylating agent mole ratio | % Conversion alkylating agent | |
|---|---|---|---|---|---|---|---|
| 11 | 1-Butene or 2-butene | 200 | 2169.76 kPa (300 psig) | 6 | 7/1 | >95 | Secondary butyl Isopropylben-zene-95% (75% *Para*, 25% *Meta*) |
| 12 | Isobutylene | 200 | 2169.76 kPa (300 psig) | 6 | 7/1 | >95 | Tertiary butyl Isopropylben-zene (95% *Para*) |

**0012514**

Cumene (isopropylbenzene) can be alkylated with butenes at 200°C. The reaction must be carried out in the liquid phase to prevent catalyst deactivation. Conversion of the olefins was >95% with 95% selectivity. The 1- and 2- butenes alkylated to produce secondary butylcumene and isobutylene gave tertiary butylcumene.

Examples 13, 14

*ortho*-Xylene was alkylated with $C_4$ olefins over HZSM-12 at 190°—200°C. The reaction conditions and products are given in Table VI.

TABLE VI
Ortho-xylene
Alkylation over HZSM-12

| Example | Alkylating agent | Temp.°C | Pressure | WHSV | Xylene/alkylating agent mole ratio | % Conversion alkylating agent | Products-% selectivity |
|---|---|---|---|---|---|---|---|
| 13 | 1-Butene or 2-butene | 200 | 2169.76 kPa (300 psig) | 6 | 7/1 | 95 | 1,2-Dimethyl-4-sec butylbenzene 96% |
| 14 | Isobutylene | 190 | 2169.76 kPa (300 psig) | 6 | 7/1 | 95 | 1,2-Dimethyl-4-t butylbenzene 97% |

0012514

**0012514**

Example 15

At 200°C and 2169.76 kPa (300 psig) *p*-xylene was alkylated with 1-butene or 2-butene to make 1,2-dimethyl-4-*sec*-butylbenzene with 96% selectivity. Conversion of the olefin was 95%.

Example 16

At 190°C *o*-xylene was alkylated with isobutylene to give 1,2-dimethyl-4-*t*-butylbenzene. The selectivity was 97% and conversion of the olefin 95%. The reaction was maintained for 48 hours without a decline in catalyst activity.

Example 17

Halogenated aromatics are difficult to alkylate because of the deactivating effect of the halogen upon the aromatic ring. However, using HZSM-12 as catalyst it was possible to non-selectively alkylate chlorobenzene with propylene in the liquid phase.

The results are shown in Table VII. This reaction was exceptional because 95% of the propylene was converted to isopropylchlorobenzene with a selectivity of 99%. This compares to published data which indicate aluminosilicate or $H_2SO_4$ catalysts gave approximately 85% conversion of the propylene. In the case of $H_2SO_4$, the published isomer distribution was 80%-*ortho*, 20%-*para*. The *meta* isomer was not found. With HZSM-12 as catalyst the isomer ratio was 60%-*para*, 20%-*meta*, 20%-*ortho*.

TABLE VII
Example 17
Chlorobenzene
Alkylation with propylene

| | |
|---|---|
| Temperature | 250°C |
| Pressure | 1825.02 kPa (250 psig) |
| WHSV chlorobenzene/propylene: | 8.1/0.25 |
| Chlorobenzene/propylene: | Mole/mole 10.6/1 |

| Reactor effluent | Wt.% | Selectivity, % |
|---|---|---|
| Lt. Ends | 0.19 | 1.59% |
| Chlorobenzene | 88.05 | — |
| *Ortho* chlorocumene | 2.14 | 17.91% |
| *Meta* chlorocumene | 2.39 | 20.00% |
| *Para* chlorocumene | 7.22 | 60.42% |
| Other | Trace | |

Feed-Wt. %: Chlorobenzene-96.6%        Propylene- 3.4%

From the foregoing Examples, it is apparent that zeolite ZSM-12 has exceptional alkylation activity and selectivity with respect to aromatic molecules, particularly with $C_3$ and higher olefins at temperatures of approximately 200°—250°C in the liquid phase.

Example 18

Benzene was alkylated with propylene in the presence of HZSM-12 catalyst to show the effect of temperature on the reaction. Keeping the benzene/propylene feed and the pressure substantially constant, the reactor temperature was varied from 100°—300°C and the reaction products analyzed for changes in composition. The parameters and results are given in Table VIII.

13

TABLE VIII
Alkylation of benzene with propylene to produce isopropylbenzene
Effect of Temperature

| Temperature,°C | 100 | 125 | 150 | 175 | 200 | 200 | 225 | 250 | 275 | 300 |
|---|---|---|---|---|---|---|---|---|---|---|
| WHSV Benzene | 15.2 | 15.2 | 15.3 | 15.2 | 15.2 | 28.8 | 28.8 | 28.8 | 28.8 | 28.8 |
| WHSV Propylene | 1.2 | 1.2 | 1.2 | 1.2 | 1.2 | 2.4 | 2.4 | 2.4 | 2.4 | 2.4 |
| Mole ratio: benzene/propylene | 6.7 | 6.7 | 6.7 | 6.7 | 6.7 | 6.7 | 6.7 | 6.7 | 6.7 | 6.7 |
| % Conversion benzene | 0.9 | 7.1 | 12.0 | 10.4 | 11.8 | 11.3 | 11.3 | 12.2 | 12.3 | 12.1 |
| % Conversion propylene | 14.9 | 59.0 | 97.9 | 98.4 | 98.1 | 98.1 | 99.4 | 98.1 | 98.0 | 97.8 |
| | | | | | | | | | | |
| para-diisopropylbenzene | — | 100 | 100 | 100 | 67.0 | 72.2 | 55.4 | 32.6 | 31.9 | 29.8 |
| Selectivity to isopropylbenzene | 86.04 | 92.23 | 97.06 | 97.64 | 96.99 | 95.74 | 94.92 | 93.20 | 91.46 | 85.89 |
| selecitvity to m+p diisopropylbenzene | — | 2.92 | 2.67 | 2.06 | 2.70 | 3.96 | 4.54 | 5.56 | 4.96 | 3.75 |
| Selectivity to n-propylbenzene | 0 | 0 | 0 | 0 | 0 | 0 | .17 | .89 | 3.22 | 9.99 |
| Material balance | 97.5 | 97.8 | 98.2 | 96.5 | 96.6 | 100.2 | 99.6 | 98.0 | 98.1 | 97.9 |

Note: Pressure in all cases 2169.76 kPa (300 psig).

Example 19

As in Example 18, benzene was again alkylated with propylene over ZSM-12 to produce isopropylbenzene, this time maintaining the temperature at 200°C and varying the pressure from 101.33—3548.7 kPa (0 to 500 psig). The results are shown in Table IX. From those results it will be noted that the selectivity to isopropylbenzene increases sharply as the pressure is increased from below 1200 kPa to above 1400 kPa. This pressure increase causes transition from the vapor phase to the liquid phase at the temperature employed, and the results emphasize the significance of operating in the liquid phase.

# TABLE IX
## Alkylation of benzene with propylene to produce isopropylbenzene
### Effect of pressure

| | | | | | | | | |
|---|---|---|---|---|---|---|---|---|
| Temperature,°C | 200 | 200 | 200 | 200 | 200 | 200 | 200 | 200 |
| Pressure, kPa (psig) | 101.33 (0) | 273.7 (25) | 446.07 (50) | 618.43 (75) | 790.8 (100) | 963.17 (125) | 790.8 (100) | 1135.54 (150) |
| WHSV Benzene | 31.0 | 31.0 | 31.0 | 31.0 | 31.0 | 31.0 | 30 | 30 |
| WHSV Propylene | 1.8 | 1.8 | 1.8 | 1.8 | 1.8 | 1.8 | 24 | 24 |
| Mole ratio: benzene/propylene | 9.5 | 9.5 | 9.5 | 9.5 | 9.5 | 9.5 | 6.6 | 6.6 |
| % Conversion benzene | 8.4 | 9.5 | 9.6 | 10.3 | 10.3 | 10.3 | 14.0 | 14.3 |
| % Conversion propylene | 92.6 | 97.0 | 97.5 | 94.8 | 95.1 | 94.8 | 97.9 | 95.4 |
| *para*-diisopropylbenzene in total DIPB | 67 | 68 | 71 | 77 | 78 | 77 | 66 | 76 |
| Selectivity to isopropylbenzene | 83.8 | 86.8 | 88.6 | 88.5 | 89.8 | 90.4 | 88.5 | 90.3 |
| Material balance | 101.6 | 101.4 | 102.6 | 100.5 | 99.7 | 99.7 | 99.1 | 102.4 |

## TABLE IX—Continued

| | | | | | | | | |
|---|---|---|---|---|---|---|---|---|
| Temperature, °C | 200 | 200 | 200 | 200 | 200 | 200 | 200 | 200 |
| Pressure, kPa (psig) | 1480.28 (200) | 1825.2 (250) | 2169.76 (300) | 2514.49 (350) | 1480.28 (200) | 2169.76 (300) | 2859.23 (400) | 3548.7 (500) |
| WHSV Benzene | 30 | 30 | 30 | 30 | 15.4 | 15.4 | 15.4 | 15.4 |
| WHSV Propylene | 2.4 | 2.4 | 2.4 | 2.4 | 1.2 | 1.2 | 1.2 | 1.2 |
| Mole ratio: benzene/propylene | | | | | | | | |
| % Conversion benzene | 6.6 | 6.6 | 6.6 | 6.6 | 6.8 | 6.8 | 6.8 | 6.8 |
| % Conversion propylene | 14.1 | 13.7 | 13.1 | 14.0 | 10.8 | 11.8 | 9.3 | 13.3 |
| | 96.8 | 97.1 | 95.7 | 96.8 | 98.3 | 48.1 | 87.7 | 97.9 |
| *para*-diisopropylene in total DIPB | 81 | 81 | 86 | 89 | 78 | 67 | 100 | 80 |
| Selectivity to isopropylbenzene | 94.0 | 94.8 | 96.3 | 96.7 | 95.7 | 97.0 | 97.9 | 96.4 |
| Material balance | 98.3 | 99.6 | 100.0 | 99.1 | 97.1 | 96.6 | 97.2 | 99.6 |

0012514

Example 20

Benzene was alkylated with isobutylene in the vapor phase with HZSM-12 catalyst at 190° and atmospheric pressure. The reaction is summarized in Table X.

TABLE X

Benzene alkylation with isobutylene-vapor phase

| Feed: | 10.1/1 benzene/isobutylene—mole/mole |
|---|---|
| Temperature: | 190°C |
| Pressure: | Atmospheric |
| WHSV: | 11.7 Benzene 0.8 isobutylene |
| Conversion, Wt.%: | 3.4% Benzene 39% isobutylene |

| Component | Wt.% In effluent |
|---|---|
| Light ends | 4.0 |
| Benzene | 90.2 |
| $C_8$ | 0.1 |
| Tertiary butylbenzene | 5.1 |
| See butylbenzene | 0.5 |
| $C_{10+}$ | 0.1 |

Example 21

Using the same reactants and catalyst as Example 20, the alkylation was carried out in the liquid phase by increasing the pressure to 2480.02 kPa (345 psig). The results are shown in Table XI.

TABLE XI

Benzene alkylation with isobutylene-liquid phase

| Feed: | 8.6/1 Benzene/isobutylene—mole/mole |
|---|---|
| Temperature: | 185°C |
| Pressure: | 2480.02 kPa (345 psig) |
| WHSV: | 9.2 Benzene 0.8 isobutylene |
| Conversion, Wt.%: | 10.9% Benzene 91.0% isobutylene |

| Component | Wt.% In effluent |
|---|---|
| Light ends | 0.7 |
| Benzene | 82.2 |
| $C_8$ | 0.1 |
| Tertiary butylbenzene | 16.7 |
| Sec. butylbenzene | 0.3 |

A comparison of Example 20 (vapor phase) with Example 21 (liquid phase) demonstrates the surprising and substantial increase in conversion and selectivity encountered by operating in the liquid phase at low temperature and in the presence of ZSM-12 catalyst.

**Claims**

1. A process for the liquid phase alkylation of an aromatic hydrocarbon compound comprising contacting such compound with an alkylating agent having from one to five carbon atoms at a temperature between 100°C and 250°C and a pressure at least sufficient to maintain a liquid phase but not greater than $2 \times 10^4$ kPa in the presence of a catalyst comprising zeolite ZSM-12.

2. A process according to claim 1 wherein said temperature is between 200°C and 250°C.

3. A process according to claim 1 or claim 2 wherein the aromatic hydrocarbon bears a non-polar substituent.

4. A process according to any preceding claim wherein the aromatic hydrocarbon bears a polar substituent.

5. A process according to any preceding claim wherein the alkylating agent is an olefin.

6. A process according to claim 5 wherein the olefin has at least three carbon atoms.

7. A process according to any of claims 1 to 5 wherein the alkylating agent is an alcohol.

8. A process according to any preceding claim wherein the zeolite is composited with a binder.

# 0 012 514

**Patentansprüche**

1. Verfahren zur Flüssigphasen-Alkylierung einer aromatischen Kohlenwasserstoffverbindung, dadurch gekennzeichnet, daß man die Verbindung mit einem Alkylierungsmittel mit 1 bis 5 Kohlenstoffatomen bei einer Temperatur zwischen 100°C und 250°C und einem Druck, der zur Aufrechterhaltung einer flüssigen Phase wenigstens ausreichend, jedoch nicht größer als $2 \times 10^4$ kPa ist, in Gegenwart eines Katalysators in Berührung bringt, der Zeolith ZSM-12 enthält.

2. Verfahren nach Anspruch 1, dadurch gekennzeichnet, daß die Temperatur zwischen 200°C und 250°C ist.

3. Verfahren nach Anspruch 1 oder 2, dadurch gekennzeichnet, daß der aromatische Kohlenwasserstoff einen nichtpolaren Substituenten trägt.

4. Verfahren nach einem der vorhergehenden Ansprüche, dadurch gekennzeichnet, daß der aromatische Kohlenwasserstoff einen polaren Substituenten trägt.

5. Verfahren nach einem der vorhergehenden Ansprüche, dadurch gekennzeichnet, daß das Alkylierungsmittel ein Olefin ist.

6. Verfahren nach Anspruch 5, dadurch gekennzeichnet, daß das Olefin wenigstens drei Kohlenstoffatome hat.

7. Verfahren nach einem der Ansprüche 1 bis 5, dadurch gekennzeichnet, daß das Alkylierungsmittel ein Alkohol ist.

8. Verfahren nach einem der vorhergehenden Ansprüche, dadurch gekennzeichnet, daß der Zeolith mit einem Bindemittel zusammengesetzt ist.

**Revendications**

1. Procédé pour l'alcoylation en phase liquide d'un composé hydrocarboné aromatique, caractérisé en ce qu'il comprend la mise en contact dudit composé avec un agent d'alcoylation contenant entre 1 et 5 atomes de carbone, à une température comprise entre 100°C et 250°C et à une pression au moins suffisante pour maintenir une phase liquide mais ne dépassant pas $2 \times 10^4$ kPa, en présence d'un catalyseur comprenant de la zéolite ZSM-12.

2. Procédé selon la revendication 1, caractérisé en ce que ladite température est comprise entre 200 et 250°C.

3. Procédé selon l'une quelconque des revendications 1 ou 2, caractérisé en ce que l'hydrocarbure aromatique porte un substituant non polaire.

4. Procédé selon l'une quelconque des revendications précédentes, caractérisé en ce que l'hydrocarbure aromatique porte un substituant polaire.

5. Procédé selon l'une quelconque des revendications précédentes, caractérisé en ce que l'agent d'alcoylation est une oléfine.

6. Procédé selon la revendication 5, caractérisé en ce que l'oléfine contient au moins 3 atomes de carbone.

7. Procédé selon l'une quelconque des revendications 1 à 5, caractérisé en ce que l'agent d'alcoylation est un alcool.

8. Procédé selon l'une quelconque des revendications précédentes, caractérisé en ce que la zéolite est combinée avec un liant.